# EUROPEAN PATENT SPECIFICATION

(11) **EP 3 298 154 B1**
(45) Date of publication and mention of the grant of the patent: **11.09.2019**
(21) Application number: 16739141.6
(22) Date of filing: 15.07.2016
(51) Int. Cl.: C12P 7/40, C12P 7/42, C12N 15/52, C12N 9/10, C12N 9/04, C12N 9/88

(54) **PRODUCTION OF 3-HYDROXYBUTYRATE**
HERSTELLUNG VON 3-HYDROXYBUTYRAT
PRODUCTION DE 3-HYDROXYBUTYRATE

(30) Priority: 29.07.2015 EP 15178769
(43) Date of publication of application: 28.03.2018
(73) Proprietor: Evonik Degussa GmbH, 45128 Essen (DE)
(72) Inventor: HAAS, Thomas, 48161 Münster (DE); HECKER, Anja, 48147 Münster (DE); PÖTTER, Markus, Shanghai 201702 (CN); BÜLTER, Thomas, 47279 Duisburg (DE)
(74) Representative: Evonik Patent Association
(86) International application number: PCT/EP2016/066875
(87) International publication number: WO 2017/016902

(56) References cited:
- EP-A1- 2 770 062
- WO-A1-03/046159
- WO-A1-2009/020279
- WO-A1-2013/012975
- WO-A1-2013/036812
- WO-A1-2013/172928
- WO-A1-2014/190251
- US-A- 4 211 846
- US-A1- 2003 203 459
- LEE S.Y. ET AL: "Metabolic engineering of Escherichia coli for production of enantiomerically pure (R)-(-)-hydroxycarboxylic acids", APPLIED AND ENVIRONMENTAL MICROBIOLOGY, vol. 69, no. 6, 1 June 2003 (2003-06-01), pages 3421-3426, XP002372566, ISSN: 0099-2240, DOI: 10.1128/AEM.69.6.3421-3426.2003
- GAO H.-J. ET AL: "Enhanced production of D-(-)-3-hydroxybutyric acid by recombinant Escherichia coli", FEMS MICROBIOLOGY LETTERS, vol. 213, no. 1, 18 June 2002 (2002-06-18) , pages 59-65, XP002583389, ISSN: 0378-1097
- LIU Q. ET AL: "Microbial production of R-3-hydroxybutyric acid by recombinant E. coli harboring genes of phbA, phbB, and tesB", APPLIED MICROBIOLOGY AND BIOTECHNOLOGY, vol. 76, no. 4, 4 July 2007 (2007-07-04), pages 811-818, XP019538724, ISSN: 1432-0614, DOI: 10.1007/S00253-007-1063-0
- LEE S.-H. ET AL: "Biosynthesis of enantiopure (S)-3-hydroxybutyric acid in metabolically engineered Escherichia coli", APPLIED MICROBIOLOGY AND BIOTECHNOLOGY, vol. 79, no. 4, 7 May 2008 (2008-05-07), pages 633-641, XP019623626, ISSN: 1432-0614
- MATSUMOTO K. ET AL: "Efficient ()-3-hydroxybutyrate production using acetyl CoA-regenerating pathway catalyzed by coenzyme A transferase", APPLIED MICROBIOLOGY AND BIOTECHNOLOGY, vol. 97, no. 1, 18 May 2012 (2012-05-18), pages 205-210, XP035158268, ISSN: 1432-0614, DOI: 10.1007/S00253-012-4104-2 -& MATSUMOTO K. ET AL: "Erratum to: Efficient ()-3-hydroxybutyrate production using acetyl CoA-regenerating pathway catalyzed by coenzyme A transferase", APPLIED MICROBIOLOGY AND BIOTECHNOLOGY, vol. 97, no. 1, 11 November 2012 (2012-11-11), page 439, XP035158252, ISSN: 1432-0614, DOI: 10.1007/S00253-012-4563-5

### SEQUENCE LISTING

<110> Evonik Industries AG
<120> PRODUCTION OF 3-HYDROXYBUTYRATE
<130> 201400344EP
<150> 15178769.4
   <151> 2015-07-29
<160> 8
<170> PatentIn version 3.5
<210> 1
   <211> 1179
   <212> DNA
   <213> Clostridium acetobutylicum
<400> 1
   atgaaagaag ttgtaatagc tagtgcagta agaacagcga ttggatctta tggaaagtct 60
   cttaaggatg taccagcagt agatttagga gctacagcta taaaggaagc agttaaaaaa 120
   gcaggaataa aaccagagga tgttaatgaa gtcattttag gaaatgttct tcaagcaggt 180
   ttaggacaga atccagcaag acaggcatct tttaaagcag gattaccagt tgaaattcca 240
   gctatgacta ttaataaggt ttgtggttca ggacttagaa cagttagctt agcagcacaa 300
   attataaaag caggagatgc tgacgtaata atagcaggtg gtatggaaaa tatgtctaga 360
   gctccttact tagcgaataa cgctagatgg ggatatagaa tgggaaacgc taaatttgtt 420
   gatgaaatga tcactgacgg attgtgggat gcatttaatg attaccacat gggaataaca 480
   gcagaaaaca tagctgagag atggaacatt tcaagagaag aacaagatga gtttgctctt 540
   gcatcacaaa aaaaagctga agaagctata aaatcaggtc aatttaaaga tgaaatagtt 600
   cctgtagtaa ttaaaggcag aaagggagaa actgtagttg atacagatga gcaccctaga 660
   tttggatcaa ctatagaagg acttgcaaaa ttaaaacctg ccttcaaaaa agatggaaca 720
   gttacagctg gtaatgcatc aggattaaat gactgtgcag cagtacttgt aatcatgagt 780
   gcagaaaaag ctaaagagct tggagtaaaa ccacttgcta agatagtttc ttatggttca 840
   gcaggagttg acccagcaat aatgggatat ggacctttct atgcaacaaa agcagctatt 900
   gaaaaagcag gttggacagt tgatgaatta gatttaatag aatcaaatga agcttttgca 960
   gctcaaagtt tagcagtagc aaaagattta aaatttgata tgaataaagt aaatgtaaat 1020
   ggaggagcta ttgcccttgg tcatccaatt ggagcatcag gtgcaagaat actcgttact 1080
   cttgtacacg caatgcaaaa aagagatgca aaaaaaggct tagcaacttt atgtataggt 1140
   ggcggacaag gaacagcaat attgctagaa aagtgctag 1179
<210> 2
   <211> 392
   <212> PRT
   <213> Clostridium acetobutylicum
<400> 2
   Met Lys Glu Val Val Ile Ala Ser Ala Val Arg Thr Ala Ile Gly Ser
   1               5                   10                  15
   Tyr Gly Lys Ser Leu Lys Asp Val Pro Ala Val Asp Leu Gly Ala Thr
               20                  25                  30
   Ala Ile Lys Glu Ala Val Lys Lys Ala Gly Ile Lys Pro Glu Asp Val
           35                  40                  45
   Asn Glu Val Ile Leu Gly Asn Val Leu Gln Ala Gly Leu Gly Gln Asn
       50                  55                  60
   Pro Ala Arg Gln Ala Ser Phe Lys Ala Gly Leu Pro Val Glu Ile Pro
   65                  70                  75                  80
   Ala Met Thr Ile Asn Lys Val Cys Gly Ser Gly Leu Arg Thr Val Ser
                   85                  90                  95
   Leu Ala Ala Gln Ile Ile Lys Ala Gly Asp Ala Asp Val Ile Ile Ala
               100                 105                 110
   Gly Gly Met Glu Asn Met Ser Arg Ala Pro Tyr Leu Ala Asn Asn Ala
           115                 120                 125
   Arg Trp Gly Tyr Arg Met Gly Asn Ala Lys Phe Val Asp Glu Met Ile
       130                 135                 140
   Thr Asp Gly Leu Trp Asp Ala Phe Asn Asp Tyr His Met Gly Ile Thr
   145                 150                 155                 160
   Ala Glu Asn Ile Ala Glu Arg Trp Asn Ile Ser Arg Glu Glu Gln Asp
                   165                 170                 175
   Glu Phe Ala Leu Ala Ser Gln Lys Lys Ala Glu Glu Ala Ile Lys Ser
               180                 185                 190
   Gly Gln Phe Lys Asp Glu Ile Val Pro Val Val Ile Lys Gly Arg Lys
           195                 200                 205
   Gly Glu Thr Val Val Asp Thr Asp Glu His Pro Arg Phe Gly Ser Thr
       210                 215                 220
   Ile Glu Gly Leu Ala Lys Leu Lys Pro Ala Phe Lys Lys Asp Gly Thr
   225                 230                 235                 240
   Val Thr Ala Gly Asn Ala Ser Gly Leu Asn Asp Cys Ala Ala Val Leu
                   245                 250                 255
   Val Ile Met Ser Ala Glu Lys Ala Lys Glu Leu Gly Val Lys Pro Leu
               260                 265                 270
   Ala Lys Ile Val Ser Tyr Gly Ser Ala Gly Val Asp Pro Ala Ile Met
           275                 280                 285
   Gly Tyr Gly Pro Phe Tyr Ala Thr Lys Ala Ala Ile Glu Lys Ala Gly
       290                 295                 300
   Trp Thr Val Asp Glu Leu Asp Leu Ile Glu Ser Asn Glu Ala Phe Ala
   305                 310                 315                 320
   Ala Gln Ser Leu Ala Val Ala Lys Asp Leu Lys Phe Asp Met Asn Lys
                   325                 330                 335
   Val Asn Val Asn Gly Gly Ala Ile Ala Leu Gly His Pro Ile Gly Ala
               340                 345                 350
   Ser Gly Ala Arg Ile Leu Val Thr Leu Val His Ala Met Gln Lys Arg
           355                 360                 365
   Asp Ala Lys Lys Gly Leu Ala Thr Leu Cys Ile Gly Gly Gly Gln Gly
       370                 375                 380
   Thr Ala Ile Leu Leu Glu Lys Cys
   385                 390
<210> 3
   <211> 657
   <212> DNA
   <213> Clostridium acetobutylicum
<400> 3
   atgaactcta aaataattag atttgaaaat ttaaggtcat tctttaaaga tgggatgaca        60
   attatgattg gaggtttttt aaactgtggc actccaacca aattaattga ttttttagtt       120
   aatttaaata taaagaattt aacgattata agtaatgata catgttatcc taatacaggt       180
   attggtaagt taatatcaaa taatcaagta aaaaagctta ttgcttcata tataggcagc       240
   aacccagata ctggcaaaaa actttttaat aatgaacttg aagtagagct ctctccccaa       300
   ggaactctag tggaaagaat acgtgcaggc ggatctggct taggtggtgt actaactaaa       360
   acaggtttag gaactttgat tgaaaaagga aagaaaaaaa tatctataaa tggaacggaa       420
   tatttgttag agctacctct tacagccgat gtagcattaa ttaaaggtag tattgtagat       480
   gaggccggaa acaccttcta taaaggtact actaaaaact ttaatcccta tatggcaatg       540
   gcagctaaaa ccgtaatagt tgaagctgaa aatttagtta gctgtgaaaa actagaaaag       600
   gaaaaagcaa tgacccccgg agttcttata aattatatag taaaggagcc tgcataa          657
<210> 4
   <211> 218
   <212> PRT
   <213> Clostridium acetobutylicum
<400> 4
   Met Asn Ser Lys Ile Ile Arg Phe Glu Asn Leu Arg Ser Phe Phe Lys
   1               5                   10                  15
   Asp Gly Met Thr Ile Met Ile Gly Gly Phe Leu Asn Cys Gly Thr Pro
               20                  25                  30
   Thr Lys Leu Ile Asp Phe Leu Val Asn Leu Asn Ile Lys Asn Leu Thr
           35                  40                  45
   Ile Ile Ser Asn Asp Thr Cys Tyr Pro Asn Thr Gly Ile Gly Lys Leu
       50                  55                  60
   Ile Ser Asn Asn Gln Val Lys Lys Leu Ile Ala Ser Tyr Ile Gly Ser
   65                  70                  75                  80
   Asn Pro Asp Thr Gly Lys Lys Leu Phe Asn Asn Glu Leu Glu Val Glu
                   85                  90                  95
   Leu Ser Pro Gln Gly Thr Leu Val Glu Arg Ile Arg Ala Gly Gly Ser
               100                 105                 110
   Gly Leu Gly Gly Val Leu Thr Lys Thr Gly Leu Gly Thr Leu Ile Glu
           115                 120                 125
   Lys Gly Lys Lys Lys Ile Ser Ile Asn Gly Thr Glu Tyr Leu Leu Glu
       130                 135                 140
   Leu Pro Leu Thr Ala Asp Val Ala Leu Ile Lys Gly Ser Ile Val Asp
   145                 150                 155                 160
   Glu Ala Gly Asn Thr Phe Tyr Lys Gly Thr Thr Lys Asn Phe Asn Pro
                   165                 170                 175
   Tyr Met Ala Met Ala Ala Lys Thr Val Ile Val Glu Ala Glu Asn Leu
               180                 185                 190
   Val Ser Cys Glu Lys Leu Glu Lys Glu Lys Ala Met Thr Pro Gly Val
           195                 200                 205
   Leu Ile Asn Tyr Ile Val Lys Glu Pro Ala
       210                 215
<210> 5
   <211> 666
   <212> DNA
   <213> Clostridium acetobutylicum
<400> 5
   atgattaatg ataaaaacct agcgaaagaa ataatagcca aaagagttgc aagagaatta      60
   aaaaatggtc aacttgtaaa cttaggtgta ggtcttccta ccatggttgc agattatata     120
   ccaaaaaatt tcaaaattac tttccaatca gaaaacggaa tagttggaat gggcgctagt     180
   cctaaaataa atgaggcaga taaagatgta gtaaatgcag gaggagacta tacaacagta     240
   cttcctgacg gcacattttt cgatagctca gtttcgtttt cactaatccg tggtggtcac     300
   gtagatgtta ctgttttagg ggctctccag gtagatgaaa agggtaatat agccaattgg     360
   attgttcctg gaaaaatgct ctctggtatg ggtggagcta tggatttagt aaatggagct     420
   aagaaagtaa taattgcaat gagacataca aataaaggtc aacctaaaat tttaaaaaaa     480
   tgtacacttc ccctcacggc aaagtctcaa gcaaatctaa ttgtaacaga acttggagta     540
   attgaggtta ttaatgatgg tttacttctc actgaaatta ataaaaacac aaccattgat     600
   gaaataaggt ctttaactgc tgcagattta ctcatatcca atgaacttag acccatggct     660
   gtttag                                                                666
<210> 6
   <211> 221
   <212> PRT
   <213> Clostridium acetobutylicum
<400> 6
   Met Ile Asn Asp Lys Asn Leu Ala Lys Glu Ile Ile Ala Lys Arg Val
   1               5                   10                  15
   Ala Arg Glu Leu Lys Asn Gly Gln Leu Val Asn Leu Gly Val Gly Leu
               20                  25                  30
   Pro Thr Met Val Ala Asp Tyr Ile Pro Lys Asn Phe Lys Ile Thr Phe
           35                  40                  45
   Gln Ser Glu Asn Gly Ile Val Gly Met Gly Ala Ser Pro Lys Ile Asn
       50                  55                  60
   Glu Ala Asp Lys Asp Val Val Asn Ala Gly Gly Asp Tyr Thr Thr Val
   65                  70                  75                  80
   Leu Pro Asp Gly Thr Phe Phe Asp Ser Ser Val Ser Phe Ser Leu Ile
                   85                  90                  95
   Arg Gly Gly His Val Asp Val Thr Val Leu Gly Ala Leu Gln Val Asp
               100                 105                 110
   Glu Lys Gly Asn Ile Ala Asn Trp Ile Val Pro Gly Lys Met Leu Ser
           115                 120                 125
   Gly Met Gly Gly Ala Met Asp Leu Val Asn Gly Ala Lys Lys Val Ile
       130                 135                 140
   Ile Ala Met Arg His Thr Asn Lys Gly Gln Pro Lys Ile Leu Lys Lys
   145                 150                 155                 160
   Cys Thr Leu Pro Leu Thr Ala Lys Ser Gln Ala Asn Leu Ile Val Thr
                   165                 170                 175
   Glu Leu Gly Val Ile Glu Val Ile Asn Asp Gly Leu Leu Leu Thr Glu
               180                 185                 190
   Ile Asn Lys Asn Thr Thr Ile Asp Glu Ile Arg Ser Leu Thr Ala Ala
           195                 200                 205
   Asp Leu Leu Ile Ser Asn Glu Leu Arg Pro Met Ala Val
       210                 215                 220
<210> 7
   <211> 1056
   <212> DNA
   <213> Clostridium beijerinckii
<400> 7
   atgaaaggtt ttgcaatgct aggtattaat aagttaggat ggatcgaaaa agaaaggcca      60
   gttgcgggtt catatgatgc tattgtacgc ccattagcag tatctccgtg tacatcagat     120
   atacatactg tttttgaggg agctcttgga gataggaaga atatgatttt agggcatgaa     180
   gctgtaggtg aagttgttga agtaggaagt gaagtgaagg attttaaacc tggtgacaga     240
   gttatagttc cttgtacaac tccagattgg agatctttgg aagttcaagc tggttttcaa     300
   cagcactcaa acggtatgct cgcaggatgg aaattttcaa atttcaagga tggagttttt     360
   ggtgaatatt ttcatgtaaa tgatgcggat atgaatcttg cgattctacc taaagacatg     420
   ccattagaaa atgctgttat gataacagat atgatgacta ctggatttca tggagcagaa     480
   cttgcagata ttcaaatggg ttcaagtgtt gtggtaattg gcattggagc tgttggctta     540
   atgggaatag caggtgctaa attacgtgga gcaggtagaa taattggagt ggggagcagg     600
   ccgatttgtg ttgaggctgc aaaattttat ggagcaacag atattctaaa ttataaaaat     660
   ggtcatatag ttgatcaagt tatgaaatta acgaatggaa aaggcgttga ccgcgtaatt     720
   atggcaggcg gtggttctga aacattatcc caagcagtat ctatggttaa accaggagga     780
   ataatttcta atataaatta tcatggaagt ggagatgctt tactaatacc acgtgtagaa     840
   tggggatgtg gaatggctca caagactata aaaggaggtc tttgtcctgg gggacgtttg     900
   agagcagaaa tgttaagaga tatggtagta tataatcgtg ttgatctaag taaattagtt     960
   acacatgtat atcatggatt tgatcacata gaagaagcac tgttattaat gaaagacaag    1020
   ccaaaagact taattaaagc agtagttata ttataa                              1056
<210> 8
   <211> 351
   <212> PRT
   <213> Clostridium beijerinckii
<400> 8
   Met Lys Gly Phe Ala Met Leu Gly Ile Asn Lys Leu Gly Trp Ile Glu
   1               5                   10                  15
   Lys Glu Arg Pro Val Ala Gly Ser Tyr Asp Ala Ile Val Arg Pro Leu
               20                  25                  30
   Ala Val Ser Pro Cys Thr Ser Asp Ile His Thr Val Phe Glu Gly Ala
           35                  40                  45
   Leu Gly Asp Arg Lys Asn Met Ile Leu Gly His Glu Ala Val Gly Glu
       50                  55                  60
   Val Val Glu Val Gly Ser Glu Val Lys Asp Phe Lys Pro Gly Asp Arg
   65                  70                  75                  80
   Val Ile Val Pro Cys Thr Thr Pro Asp Trp Arg Ser Leu Glu Val Gln
                   85                  90                  95
   Ala Gly Phe Gln Gln His Ser Asn Gly Met Leu Ala Gly Trp Lys Phe
               100                 105                 110
   Ser Asn Phe Lys Asp Gly Val Phe Gly Glu Tyr Phe His Val Asn Asp
           115                 120                 125
   Ala Asp Met Asn Leu Ala Ile Leu Pro Lys Asp Met Pro Leu Glu Asn
       130                 135                 140
   Ala Val Met Ile Thr Asp Met Met Thr Thr Gly Phe His Gly Ala Glu
   145                 150                 155                 160
   Leu Ala Asp Ile Gln Met Gly Ser Ser Val Val Val Ile Gly Ile Gly
                   165                 170                 175
   Ala Val Gly Leu Met Gly Ile Ala Gly Ala Lys Leu Arg Gly Ala Gly
               180                 185                 190
   Arg Ile Ile Gly Val Gly Ser Arg Pro Ile Cys Val Glu Ala Ala Lys
           195                 200                 205
   Phe Tyr Gly Ala Thr Asp Ile Leu Asn Tyr Lys Asn Gly His Ile Val
       210                 215                 220
   Asp Gln Val Met Lys Leu Thr Asn Gly Lys Gly Val Asp Arg Val Ile
   225                 230                 235                 240
   Met Ala Gly Gly Gly Ser Glu Thr Leu Ser Gln Ala Val Ser Met Val
                   245                 250                 255
   Lys Pro Gly Gly Ile Ile Ser Asn Ile Asn Tyr His Gly Ser Gly Asp
               260                 265                 270
   Ala Leu Leu Ile Pro Arg Val Glu Trp Gly Cys Gly Met Ala His Lys
           275                 280                 285
   Thr Ile Lys Gly Gly Leu Cys Pro Gly Gly Arg Leu Arg Ala Glu Met
       290                 295                 300
   Leu Arg Asp Met Val Val Tyr Asn Arg Val Asp Leu Ser Lys Leu Val
   305                 310                 315                 320
   Thr His Val Tyr His Gly Phe Asp His Ile Glu Glu Ala Leu Leu Leu
                   325                 330                 335
   Met Lys Asp Lys Pro Lys Asp Leu Ile Lys Ala Val Val Ile Leu
               340                 345                 350

## Description

### FIELD OF THE INVENTION

The present invention relates to a biotechnological method of producing 3-hydroxybutyrate and/or variants thereof. In particular, the method relates to a biotechnological production of 3-hydroxybutyrate from a carbon source via acetoacetyl-CoA and acetoacetate.

### BACKGROUND OF THE INVENTION

3-hydroxybutyrate also known as beta-hydroxybutyric acid is an organic compound with the formula CH₃CH(OH)CH₂CO₂H. It is a beta hydroxy acid and is a chiral compound having two enantiomers, D-3-hydroxybutyric acid and L-3-hydroxybutyric acid. Its oxidized and polymeric derivatives occur widely in nature. 3-hydroxybutyrate is the precursor to polyesters, which are biodegradable plastics including poly(3-hydroxybutyrate). 3-hydroxybutyrate is also the precursor for production of 2-hydroxyisobutyric acid and polyhydroxyalkanoates containing 2-hydroxyisobutyric acid monomer units including methacrylic acid. Methacrylic acid, its esters and polymers are widely used for producing acrylic glass panes, injection-moulded products, coatings and many other products.

There are several methods known in the art for production of 3-hydroxybutyrate. However, most of these methods use petroleum as a starting point for production of 3-hydroxybutyrate. The over-utilization of petroleum and petroleum based products has caused environmental issues including increasing atmospheric concentration of CO₂, pollution from petrochemical production and use, and disposal of non-biodegradable plastic materials. More importantly, petroleum resources are finite and not renewable in nature. For these reasons it is necessary to seek alternative approaches to produce fuels and chemicals using renewable resources. Photosynthetic cyanobacteria have attracted significant attention in recent years as a 'microbial factory' to produce biofuels and chemicals due to their capability to utilize solar energy and CO₂ as the sole energy and carbon sources, respectively. Cyanobacteria are also natural producers of the naturally-occurring biodegradable plastic poly-hydroxybutyrate (PHB). Despite efforts to enhance PHB biosynthesis through both genetic engineering strategies and the optimization of culture conditions, PHB biosynthesis by cyanobacteria was a multistage cultivation process that involved nitrogen starvation followed by supplementation of fructose or acetate, which does not capitalize on the important photosynthetic potential of cyanobacteria. Most importantly, as neither lipids nor PHB are secreted by the cells, the required processes for their extraction are energy-intensive and remain as one of the major hurdles for commercial applications.

Accordingly, there is a need in the art for a more efficient and/or an alternative biotechnological method for producing 3-hydroxybutyrate

WO 03/046159 discloses production of 3-hydroxybutyrate by genetically engineered microbial *Escherichia coli* cells comprising polyhydroxyalkanoate (PHA) biosynthesis-related enzymes from *Ralstonia eutropha,* including beta-ketothiolase (PhaA), NAPDH-dependent acetoacetyl-CoA reductase (PhaB) and PHA synthase (PhaC), as well as *R. eutropha* PHA depolymerase, wherein furthermore (R)-3-hydroxybutyrate would not be metabolized to acetoacetate in the absence of an endogenous (R)-3-hydroxybutyrate dehydrogenase.

### DESCRIPTON OF THE INVENTION

The present invention provides a cell that has been genetically modified to produce acetoacetate. In particular, the cell may be capable of converting the acetoacetate to 3-hydroxybutyrate. This is advantageous as a single cell may be used to produce 3-hydroxybutyrate, making the process simpler and requiring no steps of separation and thus no loss of material along the way. The cell according to any aspect of the present invention may also be reused reducing waste and causing an overall increased efficiency of production of 3-hydroxybutyrate. 3-hydroxybutyrate may also be produced from waste gases thus not relying on fossil fuels.

According to one aspect of the present invention, there is provided a microbial cell which is capable of producing 3-hydroxybutyrate and/or 3-hydroxybutyrate variants, wherein the cell is genetically modified to comprise an increased expression relative to its wild type cell of:
- an enzyme E₁ capable of catalysing the conversion of acetyl-CoA to acetoacetyl-CoA;
- an enzyme E₂ capable of catalysing the conversion of acetoacetyl-CoA to acetoacetate; and
- an enzyme E₃ capable of catalysing the conversion of acetoacetate to 3-hydroxybutyrate and/or variants thereof with the proviso that the genetically modified cell has reduced or no expression of acetoacetate decarboxylase (adc; EC 4.1.1.4)
wherein the 3-hydroxybutyrate variant is selected from the group consisting of (S)-3-hydroxybutyric acid, (S)-3-hydroxybutyrate ester, (R)-3-hydroxybutyric acid, (R)-3-hydroxybutyrate ester, sodium 3-hydroxybutyrate, and methyl 3-hydroxybutyrate.

The phrase "wild type" as used herein in conjunction with a cell or microorganism may denote a cell with a genome make-up that is in a form as seen naturally in the wild. The term may be applicable for both the whole cell and for individual genes. The term "wild type" therefore does not include such cells or such genes where the gene sequences have been altered at least partially by man using recombinant methods.

A skilled person would be able to use any method known in the art to genetically modify a cell or microorganism. According to any aspect of the present invention, the genetically modified cell may be genetically modified so that in a defined time interval, within 2 hours, in particular within 8 hours or 24 hours, it forms at least twice, especially at least 10 times, at least 100 times, at least 1000 times or at least 10000 times more acetoacetate and/or 3-hydroxybutyrate than the wild-type cell. The increase in product formation can be determined for example by cultivating the cell according to any aspect of the present invention and the wild-type cell each separately under the same conditions (same cell density, same nutrient medium, same culture conditions) for a specified time interval in a suitable nutrient medium and then determining the amount of target product (3-hydroxybutyrate) in the nutrient medium.

The genetically modified cell or microorganism may be genetically different from the wild type cell or microorganism. The genetic difference between the genetically modified microorganism according to any aspect of the present invention and the wild type microorganism may be in the presence of a complete gene, amino acid, nucleotide etc. in the genetically modified microorganism that may be absent in the wild type microorganism. The genetically modified microorganism according to any aspect of the present invention comprises enzymes that enable the microorganism to produce acetoacetate and/or 3-hydroxybutyrate and/or 3-hydroxybutyrate variants, wherein the 3-hydroxybutyrate variant is selected from the group consisting of (S)-3-hydroxybutyric acid, (S)-3-hydroxybutyrate ester, (R)-3-hydroxybutyric acid, (R)-3-hydroxybutyrate ester, sodium 3-hydroxybutyrate, and methyl 3-hydroxybutyrate. The wild type microorganism relative to the genetically modified microorganism of the present invention may have none or no detectable activity of the enzymes that enable the genetically modified microorganism to produce the acetoacetate and/or 3-hydroxybutyrate and/or variants thereof. As used herein, the term 'genetically modified microorganism' may be used interchangeably with the term 'genetically modified cell'. The genetic modification according to any aspect of the present invention is carried out on the cell of the microorganism.

The cells according to any aspect of the present invention are genetically transformed according to any method known in the art. In particular, the cells may be produced according to the method disclosed in WO/2009/077461.

The phrase 'the genetically modified cell has an increased activity, in comparison with its wild type, in enzymes' as used herein refers to the activity of the respective enzyme that is increased by a factor of at least 2, in particular of at least 10, more in particular of at least 100, yet more in particular of at least 1000 and even more in particular of at least 10000. In one example, the increased expression of an enzyme according to any aspect of the present invention may be 5, 10, 15, 20, 25, 30, 25, 40, 45, 50, 55, 60, 65, 70, 75, 80, 85, 90, 95 or 100% more relative to the expression of the enzyme in the wild type cell. Similarly, the decreased expression of an enzyme according to any aspect of the present invention may be 5, 10, 15, 20, 25, 30, 25, 40, 45, 50, 55, 60, 65, 70, 75, 80, 85, 90, 95 or 100% less relative to the expression of the enzyme in the wild type cell.

The phrase "increased activity of an enzyme", as used herein is to be understood as increased intracellular activity. Basically, an increase in enzymatic activity can be achieved by increasing the copy number of the gene sequence or gene sequences that code for the enzyme, using a strong promoter or employing a gene or allele that codes for a corresponding enzyme with increased activity and optionally by combining these measures. Genetically modified cells used in the method according to the invention are for example produced by transformation, transduction, conjugation or a combination of these methods with a vector that contains the desired gene, an allele of this gene or parts thereof and a vector that makes expression of the gene possible. Heterologous expression is in particular achieved by integration of the gene or of the alleles in the chromosome of the cell or an extrachromosomally replicating vector. In one example, 'increased activity of an enzyme' in a cell may refer to an overexpression of the enzyme in the cell.

The microorganism according to any aspect of the present invention may be an acetogenic bacterium. The term "acetogenic bacteria" as used herein refers to a microorganism which is able to perform the Wood-Ljungdahl pathway and thus is able to convert CO, CO₂ and/or hydrogen to acetate. These microorganisms include microorganisms which in their wild-type form do not have a Wood-Ljungdahl pathway, but have acquired this trait as a result of genetic modification. Such microorganisms include but are not limited to *E. coli* cells. These microorganisms may be also known as carboxydotrophic bacteria. Currently, 21 different genera of the acetogenic bacteria are known in the art (Drake et al., 2006), and these may also include some clostridia (Drake & Kusel, 2005). These bacteria are able to use carbon dioxide or carbon monoxide as a carbon source with hydrogen as an energy source (Wood, 1991). Further, alcohols, aldehydes, carboxylic acids as well as numerous hexoses may also be used as a carbon source (Drake et al., 2004). The reductive pathway that leads to the formation of acetate is referred to as acetyl-CoA or Wood-Ljungdahl pathway. In particular, the acetogenic bacteria may be selected from the group consisting of *Acetoanaerobium notera (ATCC 35199), Acetonema longum (DSM 6540), Acetobacterium carbinolicum (DSM 2925), Acetobacterium malicum (DSM 4132), Acetobacterium species no. 446 (*Morinaga et al., 1990, J. Biotechnol., Vol. 14, p. 187-194*),Acetobacterium wieringae (DSM 1911), Acetobacterium woodii (DSM 1030), Alkalibaculum bacchi (DSM 22112), Archaeoglobus fulgidus (DSM 4304), Blautia producta (DSM 2950, formerly Ruminococcus productus, formerly Peptostreptococcus productus), Butyribacterium methylotrophicum (DSM 3468), Clostridium aceticum (DSM 1496), Clostridium autoethanogenum (DSM 10061, DSM 19630 and DSM 23693), Clostridium carboxidivorans (DSM 15243), Clostridium coskatii (ATCC no. PTA-10522), Clostridium drakei (ATCC BA-623), Clostridium formicoaceticum (DSM 92), Clostridium glycolicum (DSM 1288), Clostridium Ijungdahlii (DSM 13528), Clostridium Ijungdahlii C-01 (ATCC 55988), Clostridium Ijungdahlii ERI-2 (ATCC 55380), Clostridium Ijungdahlii O-52 (ATCC 55989), Clostridium mayombei (DSM 6539), Clostridium methoxybenzovorans (DSM 12182), Clostridium ragsdalei (DSM 15248), Clostridium scatologenes (DSM 757), Clostridium species ATCC 29797 (*Schmidt et al., 1986, Chem. Eng. Commun., Vol. 45, p. 61-73*), Desulfotomaculum kuznetsovii (DSM 6115), Desulfotomaculum thermobezoicum subsp. thermosyntrophicum (DSM 14055), Eubacterium limosum (DSM 20543), Methanosarcina acetivorans C2A (DSM 2834), Moorella sp. HUC22-1 (*Sakai et al., 2004, Biotechnol. Let., Vol. 29, p. 1607-1612*), Moorella thermoacetica (DSM 521, formerly Clostridium thermoaceticum), Moorella thermoautotrophica (DSM 1974), Oxobacter pfennigii (DSM 322), Sporomusa aerivorans (DSM 13326), Sporomusa ovata (DSM 2662), Sporomusa silvacetica (DSM 10669), Sporomusa sphaeroides (DSM 2875), Sporomusa termitida (DSM 4440) and Thermoanaerobacter kivui (DSM 2030, formerly Acetogenium kivui).*

More in particular, the strain ATCC BAA-624 of *Clostridium carboxidivorans* may be used. Even more in particular, the bacterial strain labelled "P7" and "P11" of *Clostridium carboxidivorans* as described for example in U.S. 2007/0275447 and U.S. 2008/0057554 may be used.

Another particularly suitable bacterium may be *Clostridium Ijungdahlii.* In particular, strains selected from the group consisting of *Clostridium Ijungdahlii* PETC, *Clostridium Ijungdahlii* ERI2, *Clostridium Ijungdahlii* COL and *Clostridium Ijungdahlii* 0-52 may be used in the conversion of synthesis gas to hexanoic acid. These strains for example are described in WO 98/00558, WO 00/68407, ATCC 49587, ATCC 55988 and ATCC 55989.

In another example, the strain *Clostridium autoethanogenum (DSM 10061, DSM 19630 and DSM* 23693) may be used. In particular, the strain may be *Clostridium autoethanogenum DSM 10061.*

3-hydroxybutyrate (3HB) and variants thereof include (S)-3-hydroxybutyric acid, (S)-3-hydroxybutyrate ester, (R)-3-hydroxybutyric acid, (R)-3-hydroxybutyrate ester, sodium 3-hydroxybutyrate, and methyl 3-hydroxybutyrate.

The cell according to any aspect of the present invention, may produce at least acetoacetate. The acetoacetate may then be converted to 3HB. However, the final result always comprises a small percentage of acetoacetate that has not been converted to 3HB. Accordingly, the cell according to any aspect of the present invention may be capable of producing acetoacetate and/or 3HB.

The cell according to any aspect of the present invention, wherein E₁ may be a thiolase (E.C.2.3.1.9), E₂ may be an acetoacetate CoA transferase (EC 2.8.3.8) and/or E₃ may be a secondary alcohol dehydrogenase (EC 1.1.1.1). The combination of these enzymes in the cell according to any aspect of the present invention allows for a 'pull' towards the formation of the final desired product, 3-hydroxybutyrate and/or variants thereof from the starting carbon source, wherein the 3-hydroxybutyrate variant is selected from the group consisting of (S)-3-hydroxybutyric acid, (S)-3-hydroxybutyrate ester, (R)-3-hydroxybutyric acid, (R)-3-hydroxybutyrate ester, sodium 3-hydroxybutyrate, and methyl 3-hydroxybutyrate. In particular, the cell according any aspect of the present invention may lead to the hydrolysis of acetoacetyl-CoA with a high reaction rate at low substrate concentrations and therefore can prevent accumulation of acetoacetyl-CoA and establish a "pull" on the preceding, thiolase mediated reversible acetoacetyl-CoA biosynthesis reaction.

In particular, E₁ may be capable of catalyzing the conversion of acetyl-CoA to acetoacetyl-CoA. E₁ may be an acetoacetyl-CoA thiolase also known as an acetyl-Coenzyme A acetyltransferase. Acetoacetyl-CoA thiolase enzymes include the gene products of atoB from *E. coli* (Martin et al., 2003) with accession number NP_416728, thiolase derived from C. *acetobutylicum.* More in particular, E₁ may comprise an amino acid sequence that has 50, 55, 60, 65, 70, 75, 80, 85, 90, 95 or 100% sequence identity to SEQ ID NO: 2. Even more in particular, the cell according to any aspect of the present invention may be genetically modified to comprise the sequence of SEQ ID NO:2.

A skilled person may be capable of identifying other thiolases that may play the role of E₁. In particular, the a skilled person may be capable of assessing whether a functionally equivalent variant has substantially the same function as the nucleic acid or polypeptide of which it is a variant using any number of known methods. In one example, the methods outlined in Wiesenborn et al 1988, Wiesenborn et al, 1989, Peterson and Bennet, 1990, Ismail et al., 1993, de la Plaza et al, 2004 or may be used to assess the enzyme activity of E₁.

E₂ may be an acetoacetate CoA transferase (EC 2.8.3.9). The acetoacetate CoA transferase conserves the energy stored in the CoA-ester bond. These enzymes either naturally exhibit the desired acetoacetyl-CoA transferase activity or they can be engineered via directed evolution to accept acetetoacetyl-CoA as a substrate with increased efficiency. In particular, such enzymes, may also be capable of catalyzing the conversion of 3-hydroxybutyryl-CoA to 3-hydroxybutyrate via a transferase mechanism. Examples of E₂ may include the CoA transferase from *E. coli* with accession number P76459.1 or P76458.1 (Hanai et al., 2007), ctfAB from *C.acetobutylicum* with accession number NP_149326.1 or NP_149327.1 (Jojima et al., 2008), ctfAB from *Clostridium saccharoperbutylacetonicum* with accession number AAP42564.1 or AAP42565.1 (Kosaka et al., 2007) and the like. In particular, E₂ may also be selected from the group consisting of the gene products of *catl, cat2,* and *cat3* of *Clostridium kluyveri* with accession number P38946.1, P38942.2, and EDK35586.1 respectively (Seedorf et al., 2008; Sohling and Gottschalk, 1996), transferase products of *Trichomonas vaginalis* with accession number XP_001330176 (van Grinsven et al., 2008), *Trypanosoma brucei* with accession number XP_828352 (Riviere et al, 2004), *Fusobacterium nucleatum* (Barker et al., 1982), *Clostridium SB4* (Barker et al., 1978), *Clostridium acetobutylicum* (Wiesenborn et al., 1989), FN0272 and FN0273 with accession numbers NP_603179.1 and NP_603180.1 respectively (Kapatral et al., 2002), homologs in *Fusobacterium nucleatum* such as FN1857 and FN1856 with accession numbers NP_602657.1 and NP_602656.1 (Kreimeyer, et al., 2007), transferase products of *Porphyrmonas gingivalis* with accession number NP_905281.1 or NP_905290.1 and *Thermoanaerobacter tengcongensis* with accession number NP_622378.1 or NP_622379.1 (Kreimeyer, et al., 2007). More in particular, E₂ may comprise an amino acid sequence that has 50, 55, 60, 65, 70, 75, 80, 85, 90, 95 or 100% sequence identity to SEQ ID NO: 4 or 6. In particular, E₂ may comprise an amino acid sequence SEQ ID NO: 4 and 6. E₂ may comprise a nucleotide sequence that has 50, 55, 60, 65, 70, 75, 80, 85, 90, 95 or 100% sequence identity to SEQ ID NO: 3 or 5. More in particular, E₂ may comprise a nucleotide sequence that has 50, 55, 60, 65, 70, 75, 80, 85, 90, 95 or 100% sequence identity to SEQ ID NO: 3 and 5.

The expression of E₂ may be measured using any method known in the art. In particular, the increase in expression of E₂ may be measured by determining the amount of final product obtained in the presence of the enzyme and comparing the result to the amount of final product obtained in the absence of the enzyme E₂. In one example the final product may be a 3-hydroxybutyrate. In another example, the expression of E₂ may be determined by determining the amount of E₂ protein expressed in the resulting medium. In one example the expression of E₂ may be measured using the method disclosed in Charrier C., 2006.

E₃ may be an alcohol dehydrogenase. "Alcohol dehydrogenases" may include alcohol dehydrogenases which are capable of catalysing the conversion of ketones (such as acetone) to secondary alcohols (such as isopropanol), or vice versa. Such alcohol dehydrogenases include secondary alcohol dehydrogenases and primary alcohol dehydrogenases. A "secondary alcohol dehydrogenase" is one which can convert ketones (such as acetone) to secondary alcohols (such as isopropanol), or vice versa. A "primary alcohol dehydrogenase" is one which can convert aldehydes to primary alcohols, or vice versa; however, a number of primary alcohol dehydrogenases are also capable of catalysing the conversion of ketones to secondary alcohols, or vice versa. These alcohol dehydrogenases may also be referred to as "primary-secondary alcohol dehydrogenases". Membrane-bound, flavin-dependent alcohol dehydrogenases of the *Pseudomonas putida* GPO1 AlkJ type exist which use flavor cofactors instead of NAD+. A further group comprises iron-containing, oxygen-sensitive alcohol dehydrogenases which are found in bacteria and in inactive form in yeast. Another group comprises NAD+-dependent alcohol dehydrogenases, including zinc-containing alcohol dehydrogenases, in which the active center has a cysteine-coordinated zinc atom, which fixes the alcohol substrate. In one example, under the expression "alcohol dehydrogenase", as used herein, it is understood to mean an enzyme which oxidizes an aldehyde or ketone to the corresponding primary or secondary alcohol. In particular, the alcohol dehydrogenase according to any aspect of the present invention may be an NAD+-dependent alcohol dehydrogenase, i.e. an alcohol dehydrogenase which uses NAD+ as a cofactor for oxidation of the alcohol or NADH for reduction of the corresponding aldehyde or ketone. In the most preferred embodiment, the alcohol dehydrogenase is an NAD+-dependent, zinc-containing alcohol dehydrogenase. Examples of suitable NAD+-dependent alcohol dehydrogenases may include the alcohol dehydrogenase A from *Rhodococcus ruber* (database code AJ491307.1) or a variant thereof. Further examples comprising the alcohol dehydrogenases of *Ralstonia eutropha* (ACB78191.1), *Lactobacillus brevis* (YP_795183.1), *Lactobacillus kefiri* (ACF95832.1), from horse liver, of *Paracoccus pantotrophus* (ACB78182.1) and *Sphingobium yanoikuyae* (EU427523.1) and also the respective variants thereof. In one example, the expression "NAD(P)+-dependent alcohol dehydrogenase", as used herein, designates an alcohol dehydrogenase which is NAD+- and/or NADP+-dependent.

In one example, E₃ may be a secondary alcohol dehydrogenase or selected from other alcohol dehydrogenases or equivalently aldehyde reductases and can also serve as candidates for 3-hydroxybutyraldehyde reductase. T. E₃ may be the product of the gene selected from the group consisting of adhl from *Geobacillus thermoglucosidasius* with accession number AAR91477.1 (Jeon et al., 2008), SADH from *C. beijerinckii* the alcohol dehydrogenases disclosed in Tani et al., 2000 with accession number BAB122273.1 may be used as E₃. E₃ may also be selected from the group consisting of ADH2 from *Saccharomyces cerevisiae* with accession number NP_014032.1 (Atsumi et al., 2008), yqhD from *E. coli* with accession number NP_417484.1 (Sulzenbacher et al.,2004 and Perez et al., 2008), *bdh I and bdh II from* C. *acetobutylicum* with accession number NP_349892.1 and NP_349891.1 respectively (Walter et aI., 1992), and *ADH1* from *Zymomonas mobilis* with accession number YP_162971.1 (Kinoshita et aI., 1985).

More in particular, E₃ may be a secondary alcohol dehydrogenase. Even more in particular, E₃ may be a secondary alcohol dehydrogenase from *C. beijerinckii.* E₃ may comprise an amino acid sequence that has 50, 55, 60, 65, 70, 75, 80, 85, 90, 95 or 100% sequence identity to SEQ ID NO: 8. E₃ may comprise a nucleotide sequence that has 50, 55, 60, 65, 70, 75, 80, 85, 90, 95 or 100% sequence identity to SEQ ID NO: 7.

Any accession number used in the application refers to the respective sequence from the Genbank database run by the NCBI, wherein the release referred to is the one available online on the 30^{th} March 2015.

The expression of E₃ may be measured using any method known in the art. In particular, the increase in expression of E₃ may be measured by determining the amount of final product obtained in the presence of the enzyme and comparing the result to the amount of final product obtained in the absence of the enzyme E₃. In one example the final product may be a primary and/or secondary alcohol. In another example, the expression of E₃ may be determined by determining the amount of E₃ protein expressed in the resulting medium. In one example the expression of E₃ may be measured using the method disclosed in Ismaiel, A.A. (1993).

According to any aspect of the present invention, the cell comprises enzymes E₁, E₂ and E₃. In particular, E₁ may be a thiolase (E.C.2.3.1.9), E₂ may be an acetoacetate CoA transferase and E₃ may be a secondary alcohol dehydrogenase. More in particular, E₁ may comprise amino acid sequence SEQ ID NO:2, E₂ may comprise amino acid sequence SEQ ID NO:4 or 6, and E₃ may comprise amino acid sequence SEQ ID NO:8. Even more in particular, E₁ may comprise amino acid sequence SEQ ID NO:2, E₂ may comprise amino acid sequence SEQ ID NO:4 and 6, and E₃ may comprise amino acid sequence SEQ ID NO:8. In one example, E₁ may consists of amino acid sequence SEQ ID NO:2, E₂ may consists of amino acid sequence SEQ ID NO:4 and 6, and E₃ may consists of amino acid sequence SEQ ID NO:8.

According to any aspect of the present invention, the cell comprises enzymes E₁, E₂ and E₃ and reduced or no expression of acetoacetate decarboxylase (Adc; EC 4.1.1.4). The cell according to any aspect of the invention, in order to express significantly reduced levels of adc, may be genetically modified to remove expression of adc which may be achieved by using standard recombinant DNA technology known to the person skilled in the art. The gene sequences respectively responsible for production of adc may be inactivated or partially or entirely eliminated. Thus, the cell according to any aspect of the present invention expresses reduced or undetectable levels of adc or expresses functionally inactive adc.

In one example, the cell according to any aspect of the present invention may express adc naturally and may be genetically modified to reduce the expression of adc in the cell to about 0% or undetectable levels relative to the wild type cell. In another example, the cell according to any aspect of the present invention has about 0% or undetectable levels of expression of adc in its' wild type form. In particular, when the cell according to any aspect of the present invention has undetectable expression of the enzyme adc, the activity of the secondary dehydrogenase (E₃) may be enhanced thus possibly resulting in increased production of acetoacetate and/or 3-hydroxybutyrate. The final product, acetoacetate and/or 3-hydroxybutyrate may also be found in the reaction mixture. Thus the cell according to any aspect of the present invention may result in the export and/or release of acetoacetate and/or 3-hydroxybutyrate into the aqueous medium. The cells will thus not have to go through a further step of extracting the target products and therefore possibly killing the cells in the process of doing so.

Enzymes that are encoded by nucleic acids that have 90%, 95%, 99% and in particular 100% identity to the sequences according to SEQ ID NOs: 1, 3, 5 and 7, are suitable in the method of the present invention. The "nucleotide identity" relative to SEQ ID NOs: 1, 3, 5 and 7 is determined using known methods. In general, special computer programs with algorithms are used, taking into account special requirements. Methods that may be used for determination of identity first produce the greatest agreement between the sequences to be compared. Computer programs for determination of identity comprise, but are not restricted to, the GCG software package, including GAP (Deveroy, J. et al., 1984), and BLASTP, BLASTN and FASTA (Altschul, S. et al., 1990). The BLAST program can be obtained from the National Center for Biotechnology Information (NCBI) and from other sources (BLAST Manual, Altschul S. et al., 1990).

The well-known Smith-Waterman algorithm can also be used for determining nucleotide identity.

Parameters for nucleotide comparison may comprise the following:
- Algorithm Needleman and Wunsch, 1970,
- Comparison matrix
   Matches = + 10
   Mismatches = 0
   Gap penalty = 50
   Gap length penalty = 3
The GAP program is also suitable for use with the parameters given above. These parameters are usually the default parameters in the nucleotide sequence comparison.

The culture medium to be used must be suitable for the requirements of the particular strains. Descriptions of culture media for various microorganisms are given in "Manual of Methods for General Bacteriology".

All percentages (%) are, unless otherwise specified, mass percent.

With respect to the source of substrates comprising carbon dioxide and/or carbon monoxide, a skilled person would understand that many possible sources for the provision of CO and/or CO₂ as a carbon source exist. It can be seen that in practice, as the carbon source of the present invention any gas or any gas mixture can be used which is able to supply the microorganisms with sufficient amounts of carbon, so that acetate and/or ethanol, may be formed from the source of CO and/or CO₂.

Generally for the cell of the present invention the carbon source comprises at least 50% by weight, at least 70% by weight, particularly at least 90% by weight of CO₂ and/or CO, wherein the percentages by weight - % relate to all carbon sources that are available to the cell according to any aspect of the present invention. The carbon material source may be provided.

Examples of carbon sources in gas forms include exhaust gases such as synthesis gas, flue gas and petroleum refinery gases produced by yeast fermentation or clostridial fermentation. These exhaust gases are formed from the gasification of cellulose-containing materials or coal gasification. In one example, these exhaust gases may not necessarily be produced as by-products of other processes but can specifically be produced for use with the mixed culture of the present invention.

According to any aspect of the present invention, the carbon source may be synthesis gas. Synthesis gas can for example be produced as a by-product of coal gasification. Accordingly, the microorganism according to any aspect of the present invention may be capable of converting a substance which is a waste product into a valuable resource.

In another example, synthesis gas may be a by-product of gasification of widely available, low-cost agricultural raw materials for use with the mixed culture of the present invention to produce substituted and unsubstituted organic compounds.

There are numerous examples of raw materials that can be converted into synthesis gas, as almost all forms of vegetation can be used for this purpose. In particular, raw materials are selected from the group consisting of perennial grasses such as miscanthus, corn residues, processing waste such as sawdust and the like.

In general, synthesis gas may be obtained in a gasification apparatus of dried biomass, mainly through pyrolysis, partial oxidation and steam reforming, wherein the primary products of the synthesis gas are CO, H₂ and CO₂. Usually, a portion of the synthesis gas obtained from the gasification process is first processed in order to optimize product yields, and to avoid formation of tar. Cracking of the undesired tar and CO in the synthesis gas may be carried out using lime and/or dolomite. These processes are described in detail in for example, Reed, 1981.

Mixtures of sources can be used as a carbon source.

According to any aspect of the present invention, a reducing agent, for example hydrogen may be supplied together with the carbon source. In particular, this hydrogen may be supplied when the C and/or CO₂ is supplied and/or used. In one example, the hydrogen gas is part of the synthesis gas present according to any aspect of the present invention. In another example, where the hydrogen gas in the synthesis gas is insufficient for the method of the present invention, additional hydrogen gas may be supplied.

A skilled person would understand the other conditions necessary to carry out the method of the present invention. In particular, the conditions in the container (e.g. fermenter) may be varied depending on the first and second microorganisms used. The varying of the conditions to be suitable for the optimal functioning of the microorganisms is within the knowledge of a skilled person.

According to another aspect of the present invention, there is provided a method of producing at least one 3-hydroxybutyrate and/or variants thereof, wherein the 3-hydroxybutyrate variant is selected from the group consisting of (S)-3-hydroxybutyric acid, (S)-3-hydroxybutyrate ester, (R)-3-hydroxybutyric acid, (R)-3-hydroxybutyrate ester, sodium 3-hydroxybutyrate, and methyl 3-hydroxybutyrate, the method comprising
- contacting a recombinant microbial cell according to any aspect of the present invention with a medium comprising a carbon source.

In one example, the method of the present invention may be carried out in an aqueous medium with a pH between 5 and 8, 5.5 and 7. The pressure may be between 1 and 10 bar.

The term "contacting", as used herein, means bringing about direct contact between the cell according to any aspect of the present invention and the medium comprising the carbon source. For example, the cell, and the medium comprising the carbon source may be in different compartments. On particular, the carbon source may be in a gaseous state and added to the medium comprising the cells according to any aspect of the present invention.

In particular, the aqueous medium may comprise the cells and a carbon source comprising CO and/or CO₂. More in particular, the carbon source comprising CO and/or CO₂ is provided to the aqueous medium comprising the cells in a continuous gas flow. Even more in particular, the continuous gas flow comprises synthesis gas. These gases may be supplied for example using nozzles that open up into the aqueous medium, frits, membranes within the pipe supplying the gas into the aqueous medium and the like.

The overall efficiency, alcohol productivity and/or overall carbon capture of the method of the present invention may be dependent on the stoichiometry of the CO₂, CO, and H₂ in the continuous gas flow. The continuous gas flows applied may be of composition CO₂ and H₂. In particular, in the continuous gas flow, concentration range of CO₂ may be about 10-50 %, in particular 3 % by weight and H₂ would be within 44 % to 84 %, in particular, 64 to 66.04 % by weight. In another example, the continuous gas flow can also comprise inert gases like N₂, up to a N₂ concentration of 50 % by weight. Even more in particular, the carbon source according to any aspect of the present invention may comprise 50% or more H₂.

The term 'about' as used herein refers to a variation within 20 percent. In particular, the term "about" as used herein refers to +/- 20%, more in particular, +/-10%, even more in particular, +/-5% of a given measurement or value.

A skilled person would understand that it may be necessary to monitor the composition and flow rates of the streams. Control of the composition of the stream can be achieved by varying the proportions of the constituent streams to achieve a target or desirable composition. The composition and flow rate of the stream can be monitored by any means known in the art. In one example, the system is adapted to continuously monitor the flow rates and compositions of the streams and combine them to produce a single blended substrate stream in a continuous gas flow of optimal composition, and means for passing the optimised substrate stream to the cell of of the present invention.

Microorganisms which convert CO₂ and/or CO to acetate and/or ethanol, in particular acetate, as well as appropriate procedures and process conditions for carrying out this metabolic reaction is well known in the art. Such processes are, for example described in WO9800558, WO2000014052 and WO2010115054.

The term "an aqueous solution" or "medium" comprises any solution comprising water, mainly water as solvent that may be used to keep the cell according to any aspect of the present invention, at least temporarily, in a metabolically active and/or viable state and comprises, if such is necessary, any additional substrates. The person skilled in the art is familiar with the preparation of numerous aqueous solutions, usually referred to as media that may be used to keep inventive cells, for example LB medium in the case of *E. coli,* ATCC1754-Medium may be used in the case of *C. Ijungdahlii.* It is advantageous to use as an aqueous solution a minimal medium, *i.e.* a medium of reasonably simple composition that comprises only the minimal set of salts and nutrients indispensable for keeping the cell in a metabolically active and/or viable state, by contrast to complex mediums, to avoid dispensable contamination of the products with unwanted side products. For example, M9 medium may be used as a minimal medium. The cells are incubated with the carbon source sufficiently long enough to produce the desired product, 3HB and variants thereof, wherein the 3-hydroxybutyrate variant is selected from the group consisting of (S)-3-hydroxybutyric acid, (S)-3-hydroxybutyrate ester, (R)-3-hydroxybutyric acid, (R)-3-hydroxybutyrate ester, sodium 3-hydroxybutyrate, and methyl 3-hydroxybutyrate. For example for at least 1 , 2, 4, 5, 10 or 20 hours. The temperature chosen must be such that the cells according to any aspect of the present invention remains catalytically competent and/or metabolically active, for example 10 to 42°C, preferably 30 to 40 °C, in particular, 32 to 38 °C in case the cell is a *C. Ijungdahlii* cell.

According to another aspect of the present invention, there is provided a use of the cell according to any aspect of the present invention for the production of 3-hydroxybutyrate and/or variants thereof, wherein the 3-hydroxybutyrate variant is selected from the group consisting of (S)-3-hydroxybutyric acid, (S)-3-hydroxybutyrate ester, (R)-3-hydroxybutyric acid, (R)-3-hydroxybutyrate ester, sodium 3-hydroxybutyrate, and methyl 3-hydroxybutyrate.

### BRIEF DESCRIPTION OF FIGURES

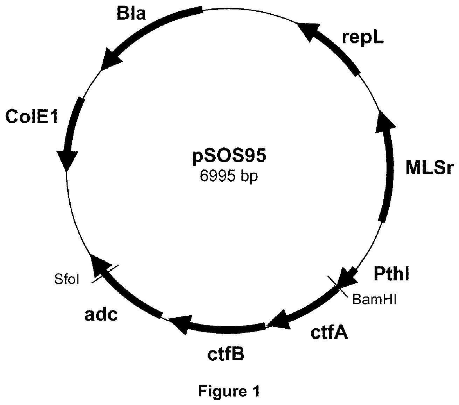

Figure 1 is an illustration of plasmid backbone pSOS95

### EXAMPLES

The foregoing describes preferred embodiments, which, as will be understood by those skilled in the art, may be subject to variations or modifications in design, construction or operation.

### Example 1

### Generation of genetically modified acetogens for the formation of 3HB via Acetoacetate

The genes Thiolase (thl) from *C. acetobutylicum* ATTC 824, Acetoacetat-transferase (ctfAB) from *C. acetobutylicum* ATTC 824 and the secondary alcohol dehydrogenase (sadh) from *C. beijerinckii* DSM 6423 were inserted into the vector pEmpty. This plasmid is based on the plasmid backbone pSOS95 (Figure 1). To use pSOS95, it was digested with BamHI and Kasl. This removes the operon ctfA-ctfB-adc, but leaves the thl promoter and the rho-independent terminator of adc. The transformation of *C. Ijungdahlii* and *C. autoethanogenum* was done as disclosed in Leang et al. 2013. The nucleotide sequences of the enzymes used are SEQ ID NOs:1, 3 (ctfA), 5 (ctfB) and 7 respectively. These sequences were transformed to be controlled by a thiolase promotor and integrated as a single operon into the vector backbone. The created vector was named pTCtS. The vector pTCts was then used to modify *C. ljungdahlii* and *C. autoethanogenum* using a method disclosed in Leang et al. 2013. The modified *C. ljungdahlii* strain was named *C. ljungdahlii* pTCtS. The modified C. *autoethanogenum* strain was named *C. autoethanogenum* pTCtS.

### Example 2

### Fermentation of 3HB strain on H₂ and CO₂ showing acetoacetate and 3HB production.

For cell culture of *C. ljungdahlii* pTCts 5 mL of the culture were anaerobically grown in 500 ml of medium (ATCC1754 medium: pH 6.0; 20 g/L MES; 1 g/L yeast extract, 0.8 g/L NaCl, 1 g/L NH₄Cl, 0.1 g/L KCI, 0.1 g/L KH₂PO₄, 0.2 g/L MgSO₄ x 7 H₂O; 0.02 g/L CaCl₂ × 2H₂O; 20 mg/L nitrilotriacetic acid 10 mg/L MnSO₄ x H₂O; 8 mg/L (NH₄)₂Fe(SO₄)₂ x 6 H₂O; 2 mg/L CoCl₂ x 6 H₂O; 2 mg/L ZnSO₄ x 7 H₂O; 0.2 mg/L CuCl₂ x 2 H₂O; 0.2 mg/L Na₂MoO₄ x 2 H₂O; 0.2 mg/L NiCl₂ x 6 H₂O; 0.2 mg/L Na₂SeO₄; 0.2 mg/L Na₂WO₄ x 2 H₂O; 20 µg/L d-Biotin, 20 µg/L folic acid, 100 g/L pyridoxine-HCI; 50 µg/L thiamine-HCI x H₂O; 50 µg/L riboflavin; 50 µg/L nicotinic acid, 50 µg/L Ca-pantothenate; 1 µg/L vitamin B12 ; 50 µg/L p-aminobenzoate; 50 µg/L lipoic acid, approximately 67.5 mg/L NaOH) with about 400 mg/L L-cysteine hydrochloride and 400 mg/L Na₂S x 9 H₂O, given 100 mg/L erythromycin). Cultivation was carried out in duplicate into 1 L glass bottles with a premixed gas mixture composed of 67% H₂, 33% CO₂ in an open water bath shaker at 37°C, 150 rpm and aeration of 3 L/h for 70.3 h.

The gas entered the medium through a filter with a pore size of 10 microns, which was mounted in the middle of the reactor, at a gassing tube. When sampling each 5 ml sample was removed for determination of OD₆₀₀ nm, pH and the product range. The determination of the product concentration was performed by semi-quantitative 1 H-NMR spectroscopy. As an internal quantification standard sodium trimethylsilylpropionate was used (T(M) SP). A culture produced 7 ppm acetoacetate and 26 ppm 3-HB in 70.3 hours. The other culture produced 24 ppm acetoacetate and 104 ppm 3-HB in 70.3 hours.

### Example 3

### Fermentation of vector control strain with no production of acetoacetate or 3HB

For cell culture of *C. ljungdahlii* pEmpty 5 mL of the culture were anaerobically grown in 500 ml of medium (ATCC1754 medium: pH 6.0; 20 g/L MES; 1 g/L yeast extract, 0.8 g/L NaCl, 1 g/L NH₄Cl, 0.1 g/L KCI, 0.1 g/L KH₂PO₄, 0.2 g/L MgSO₄ x 7 H₂O; 0.02 g/L CaCl₂ × 2H₂O; 20 mg/L nitrilotriacetic acid 10 mg/L MnSO₄ x H₂O; 8 mg/L (NH₄)₂Fe(SO₄)₂ x 6 H₂O; 2 mg/L CoCl₂ x 6 H₂O; 2 mg/L ZnSO₄ x 7 H₂O; 0.2 mg/L CuCl₂ x 2 H₂O; 0.2 mg/L Na₂MoO₄ x 2 H₂O; 0.2 mg/L NiCl₂ x 6 H₂O; 0.2 mg/L Na₂SeO₄; 0.2 mg/L Na₂WO₄ x 2 H₂O; 20 µg/L d-Biotin, 20 µg/L folic acid, 100 g/L pyridoxine-HCI; 50 µg/L thiamine-HCI x H₂O; 50 µg/L riboflavin; 50 µg/L nicotinic acid, 50 µg/L Ca-pantothenate; 1 µg/L vitamin B12 ; 50 µg/L p-aminobenzoate; 50 µg/L lipoic acid, approximately 67.5 mg/L NaOH) with about 400 mg/L L-cysteine hydrochloride and 400 mg/L Na₂S x 9 H₂O, given 100 mg/L erythromycin). Cultivation was carried out in duplicate into 1 L glass bottles with a premixed gas mixture composed of 67% H₂, 33% CO₂ in an open water bath shaker at 37°C, 150 rpm and aeration of 3 L/h for 70.3 h.

The gas entered the medium through a filter with a pore size of 10 microns, which was mounted in the middle of the reactor, at a gassing tube. When sampling each 5 ml sample was removed for determination of OD₆₀₀ nm, pH and the product range. The determination of the product concentration was performed by semi-quantitative 1 H-NMR spectroscopy. As an internal quantification standard sodium trimethylsilylpropionate was used (T(M) SP). Neither acetoacetate nor 3HB was produced.

### Example 4

### Fermentation of C. autoethanogenum pTCtS on CO, H₂ and CO₂ showing 3HB production.

For cell culture of *C. autoethanogenum* pTCts 5 mL of the culture was anaerobically grown in 500 ml of medium (ATCC1754 medium: pH 6.0; 20 g/L MES; 1 g/L yeast extract, 0.8 g/L NaCl, 1 g/L NH₄Cl, 0.1 g/L KCI, 0.1 g/L KH₂PO₄, 0.2 g/L MgSO₄ x 7 H₂O; 0.02 g/L CaCl₂ × 2H₂O; 20 mg/L nitrilotriacetic acid 10 mg/L MnSO₄ x H₂O; 8 mg/L (NH₄)₂Fe(SO₄)₂ x 6 H₂O; 2 mg/L CoCl₂ x 6 H₂O; 2 mg/L ZnSO₄ x 7 H₂O; 0.2 mg/L CuCl₂ x 2 H₂O; 0.2 mg/L Na₂MoO₄ x 2 H₂O; 0.2 mg/L NiCl₂ x 6 H₂O; 0.2 mg/L Na₂SeO₄; 0.2 mg/L Na₂WO₄ x 2 H₂O; 20 µg/L d-Biotin, 20 µg/L folic acid, 100 g/L pyridoxine-HCI; 50 µg/L thiamine-HCI x H₂O; 50 µg/L riboflavin; 50 µg/L nicotinic acid, 50 µg/L Ca-pantothenate; 1 µg/L vitamin B12 ; 50 µg/L p-aminobenzoate; 50 µg/L lipoic acid, approximately 67.5 mg/L NaOH) with about 400 mg/L L-cysteine hydrochloride and 400 mg/L Na₂S x 9 H₂O, given 100 mg/L erythromycin). Cultivation was carried out in duplicate into 1 L glass bottles with a premixed gas mixture composed of 55% CO, 20 % H₂, 10 % CO₂ and 15 % N₂ in an open water bath shaker at 37°C, 150 rpm and aeration of 3 L/h for 7 days.

The gas entered the medium through a filter with a pore size of 10 microns, which was mounted in the middle of the reactor, in a gassing tube. When sampling, 5 ml samples were removed for determination of OD₆₀₀, pH and the product range. The determination of the product concentration was performed by semi-quantitative 1 H-NMR spectroscopy. As an internal quantification standard sodium trimethylsilylpropionate was used (T(M) SP). The modified strain produced 40 ppm 3-HB in 7 days.

### Example 5

### Fermentation of C. autoethanogenum vector control strain with no production of acetoacetate or 3HB

For cell culture of *C. autoethanogenum* pEmpty 5 mL of the culture was anaerobically grown in 500 ml of medium (ATCC1754 medium: pH 6.0; 20 g/L MES; 1 g/L yeast extract, 0.8 g/L NaCl, 1 g/L NH₄Cl, 0.1 g/L KCI, 0.1 g/L KH₂PO₄, 0.2 g/L MgSO₄ x 7 H₂O; 0.02 g/L CaCl₂ × 2H₂O; 20 mg/L nitrilotriacetic acid 10 mg/L MnSO₄ x H₂O; 8 mg/L (NH₄)₂Fe(SO₄)₂ x 6 H₂O; 2 mg/L CoCl₂ x 6 H₂O; 2 mg/L ZnSO₄ x 7 H₂O; 0.2 mg/L CuCl₂ x 2 H₂O; 0.2 mg/L Na₂MoO₄ x 2 H₂O; 0.2 mg/L NiCl₂ x 6 H₂O; 0.2 mg/L Na₂SeO₄; 0.2 mg/L Na₂WO₄ x 2 H₂O; 20 µg/L d-Biotin, 20 µg/L folic acid, 100 g/L pyridoxine-HCI; 50 µg/L thiamine-HCI x H₂O; 50 µg/L riboflavin; 50 µg/L nicotinic acid, 50 µg/L Ca-pantothenate; 1 µg/L vitamin B12 ; 50 µg/L p-aminobenzoate; 50 µg/L lipoic acid, approximately 67.5 mg/L NaOH) with about 400 mg/L L-cysteine hydrochloride and 400 mg/L Na₂S x 9 H₂O, given 100 mg/L erythromycin). Cultivation was carried out in duplicate in 1 L glass bottles with a premixed gas mixture composed of 55% CO, 20 % H₂, 10 % CO₂ an open water bath shaker at 37°C, 150 rpm and aeration of 3 L/h for 7 days.

The gas entered the medium through a filter with a pore size of 10 microns, which was mounted in the middle of the reactor, in a gassing tube. When sampling, 5 ml samples were removed for determination of OD₆₀₀ nm, pH and the product range. The determination of the product concentration was performed by semi-quantitative 1 H-NMR spectroscopy. As an internal quantification standard sodium trimethylsilylpropionate was used (T(M) SP). Neither acetoacetate nor 3HB was produced.

### REFERENCES

Altschul S. et al., 1990, BLAST Manual,
Atsumi et al., Nature, 2008 451.7174:86-89
Barker et al., J. Bacteriol. 1982, 152(I):201-7
Barker et al., Biol. Chem. 1978, 253(4): 1219-25
Charrier C., Microbiology, 2006, 152: 179-185
de la Plaza et al FEMS Microbiol Lett. 2004 238: 367-374
Deveroy, J. et al., Nucleic Acid Research 12 (1984), Seite 387, Genetics Computer Group University of Wisconsin, Medicine (Wi)
Hanai et al., Appl Environ Microbiol, 2007, 73:7814-7818
Ismail et al. J Bacteriol 1993, 175: 5097-5105
Jeon et al., Biotechnol. 2008, 135.2:127-133
Jojima et al., Appl Microbiol Biotechnol, 2008 77: 1219-1224
Kapatral et al., Bact. 2002, 184(7) 2005-2018
Khorkin et a, J Mol Biol. 1998, 22: 278(5): 967-981
Kinoshita et al., Appl. Microbiol. Biotchenol. 1985, 22:249-254
Kosaka et al., Bio sci. Biotechnol Biochem. 2007, 71:58-68
Kreimeyer, et al., Biol. Chem. 2007, 282 (10) 7191-7197
Martin et al, Nat. Biotechnol. 2003, 21.7:796-802
Perez et al., Biol. Chem. 2008, 283.12:7346-7353
Peterson and Bennet, Appl Environ Microbiol. 1990 56: 3491-3498
Riviere et al.,Biol. Chem. 2004, 279:45337-45346
Seedorf et al., Proc. Natl. Acad. Sci. USA 2008, 105:2128-2133
Sohling and Gottschalk, J Bacteriol, 1996 178:871-880
Sulzenbacher et al., Mol. Biol. 2004, 342.2:489-502
Tani et al.,.Appl. Environ. Microbiol. 2000, 66.12:5231-5335
van Grinsven et al., /. Biol. Chem. 2008 283: 1411-1418
Walter et al., Bacteriol. 1992, 174.22:7149;7158
Wiesenborn et al Appl Environ Microbiol. 1988, 54: 2717-2722
Wiesenborn et al., Appl Environ Microbiol. 1989, 55:323-9
WO9800558, WO2000014052, WO2010115054,

## Claims

1. A microbial cell which is capable of producing acetoacetate, 3-hydroxybutyrate and/or 3-hydroxybutyrate variants, wherein the cell is genetically modified to comprise an increased expression relative to its wild type cell of:
- an enzyme E₁ capable of catalysing the conversion of acetyl-CoA to acetoacetyl-CoA;
- an enzyme E₂ capable of catalysing the conversion of acetoacetyl-CoA to acetoacetate; and
- an enzyme E₃ capable of catalysing the conversion of acetoacetate to 3-hydroxybutyrate and/or variants thereof and wherein the genetically modified cell has reduced or no expression of acetoacetate decarboxylase (Adc; EC 4.1.1.4)
wherein the 3-hydroxybutyrate variant is selected from the group consisting of (S)-3-hydroxybutyric acid, (S)-3-hydroxybutyrate ester, (R)-3-hydroxybutyric acid, (R)-3-hydroxybutyrate ester, sodium 3-hydroxybutyrate, and methyl 3-hydroxybutyrate.

2. The cell according to claim 1, wherein E₁ is a thiolase, E₂ is an acetoacetate CoA transferase and/or E₃ is a secondary alcohol dehydrogenase.

3. The cell according to either claim 1 or 2, wherein the thiolase (E₁) is from *C*. *acetobutylicum.*

4. The cell according to any of the preceding claims, wherein the acetoacetate CoA transferase (E₂) is from *C*. *acetobutylicum.*

5. The cell according to any of the preceding claims, wherein the secondary alcohol dehydrogenase (E₃) is from *C. beijerinckii.*

6. The cell according to any of the preceding claims, wherein
- E₁ comprises 60% sequence identity with SEQ ID NO: 2,
- E₂ comprises 60% sequence identity with SEQ ID NO: 4 or 6 and/or
- E₃ comprises 60% sequence identity with SEQ ID NO: 8.

7. The cell according to any of the preceding claims, wherein
- E₁ comprises SEQ ID NO: 2,
- E₂ comprises SEQ ID NO: 4 and 6 and
- E₃ comprises SEQ ID NO: 8.

8. The cell according to any one of the preceding claims, wherein the cell is an acetogenic cell.

9. The cell according to claim 8, wherein the acetogenic cell is selected from the group consisting of *Clostridium autothenogenum DSMZ 19630, Clostridium ragsdahlei ATCC no. BAA-622, Clostridium autoethanogenum, Moorella sp HUC22-1, Moorella thermoaceticum, Moorella thermoautotrophica, Rumicoccus productus, Acetoanaerobum, Oxobacter pfennigii, Methanosarcina barkeri, Methanosarcina acetivorans, Carboxydothermus, Desulfotomaculum kutznetsovii, Pyrococcus, Peptostreptococcus, Butyribacterium methylotrophicum ATCC 33266, Clostridium formicoaceticum, Clostridium butyricum, Lactobacillus delbrukii, Propionibacterium acidoproprionici, Proprionispera arboris, Anaerobierspirillum succiniproducens, Bacterioides amylophilus, Becterioides ruminicola, Thermoanaerobacter kivui, Acetobacterium woodii, Acetoanaerobium notera, Clostridium aceticum, Butyribacterium methylotrophicum, Moorella thermoacetica, Eubacterium limosum, Peptostreptococcus productus, Clostridium Ijungdahlii, Clostridium ATCC* 29797 and *Clostridium carboxidivorans.*

10. The cell according to any one of the preceding claims, wherein the cell is *Clostridium Ijungdahlii* or *Clostridium autothenogenum* DSMZ *10061.*

11. A method of producing acetoacetate, 3-hydroxybutyrate and/or 3-hydroxybutyrate variants, the method comprising
- contacting a recombinant microbial cell according to any one of claims 1 to 10 with a medium comprising a carbon source
wherein the 3-hydroxybutyrate variant is selected from the group consisting of (S)-3-hydroxybutyric acid, (S)-3-hydroxybutyrate ester, (R)-3-hydroxybutyric acid, (R)-3-hydroxybutyrate ester, sodium 3-hydroxybutyrate, and methyl 3-hydroxybutyrate.

12. The method according to claim 11, wherein the carbon source comprises CO₂ and/or CO.

13. The method according to either claim 11 or 12, wherein the carbon source comprises 50% or more H₂.

14. Use of the cell according to any one of the claims 1 to 10 for the production of acetoacetate, 3-hydroxybutyrate and/or 3-hydroxybutyrate variants, wherein the 3-hydroxybutyrate variant is selected from the group consisting of (S)-3-hydroxybutyric acid, (S)-3-hydroxybutyrate ester, (R)-3-hydroxybutyric acid, (R)-3-hydroxybutyrate ester, sodium 3-hydroxybutyrate, and methyl 3-hydroxybutyrate.

## Patentansprüche

1. Mikrobenzelle, die in der Lage ist, Acetoacetat, 3-Hydroxybutyrat und/oder 3-Hydroxybutyrat-Varianten zu produzieren, wobei die Zelle gentechnisch verändert ist, so dass sie relativ zu ihrer Wildtypzelle eine erhöhte Expression
- eines Enzyms E₁ mit der Fähigkeit zur Katalyse der Umwandlung von Acetyl-CoA in Acetoacetyl-CoA;
- eines Enzyms E₂ mit der Fähigkeit zur Katalyse der Umwandlung von Acetoacetyl-CoA in Acetoacetat; und
- eines Enzyms E₃ mit der Fähigkeit zur Katalyse der Umwandlung von Acetoacetat in 3-Hydroxybutyrat und/oder Varianten davon umfasst, und wobei die gentechnisch veränderte Zelle eine reduzierte oder keine Expression von Acetoacetat-Decarboxylase (Adc; EC 4.1.1.4) aufweist,
wobei die 3-Hydroxybutyrat-Variante aus der Gruppe bestehend aus (S)-3-Hydroxybuttersäure, (S)-3-Hydroxybutyrat-Ester, (R)-3-Hydroxybuttersäure, (R)-3-Hydroxybutyrat-Ester, Natrium-3-hydroxybutyrat und 3-Hydroxybuttersäuremethylester ausgewählt ist.

2. Zelle nach Anspruch 1, wobei E₁ eine Thiolase, E₂ eine Acetoacetat-CoA-Transferase und/oder E₃ eine Sekundärer-Alkohol-Dehydrogenase ist.

3. Zelle nach entweder Anspruch 1 oder 2, wobei die Thiolase (E₁) aus *C*. *acetobutylicum* stammt.

4. Zelle nach einem der vorhergehenden Ansprüche, wobei die Acetoacetat-CoA-Transferase (E2) aus *C. acetobutylicum* stammt.

5. Zelle nach einem der vorhergehenden Ansprüche, wobei die Sekundärer-Alkohol-Dehydrogenase (E3) aus *C. beijerinckii* stammt.

6. Zelle nach einem der vorhergehenden Ansprüche, wobei
- E₁ 60% Sequenzidentität mit SEQ ID NO: 2 umfasst,
- E₂ 60% Sequenzidentität mit SEQ ID NO: 4 oder 6 umfasst und/oder
- E₃ 60% Sequenzidentität mit SEQ ID NO: 8 umfasst.

7. Zelle nach einem der vorhergehenden Ansprüche, wobei
- E₁ SEQ ID NO: 2 umfasst,
- E₂ SEQ ID NO: 4 und 6 umfasst und
- E₃ SEQ ID NO: 8 umfasst.

8. Zelle nach einem der vorhergehenden Ansprüche, wobei es sich bei der Zelle um eine acetogene Zelle handelt.

9. Zelle nach Anspruch 8, wobei die acetogene Zelle ausgewählt ist aus der Gruppe bestehend aus *Clostridium autothenogenum DSMZ 19630, Clostridium ragsdahlei ATCC no. BAA-622, Clostridium autoethanogenum, Moorella sp HUC22-1, Moorella thermoaceticum, Moorella thermoautotrophica, Rumicoccus productus, Acetoanaerobum*, *Oxobacter pfennigii, Methanosarcina barkeri, Methanosarcina acetivorans, Carboxydothermus, Desulfotomaculum kutznetsovii, Pyrococcus, Peptostreptococcus, Butyribacterium methylotrophicum ATCC 33266, Clostridium formicoaceticum, Clostridium butyricum, Lactobacillus delbrukii, Propionibacterium acidoproprionici, Proprionispera arboris, Anaerobierspirillum succiniproducens, Bacterioides amylophilus, Becterioides ruminicola, Thermoanaerobacter kivui, Acetobacterium woodii, Acetoanaerobium notera, Clostridium aceticum, Butyribacterium methylotrophicum, Moorella thermoacetica, Eubacterium limosum, Peptostreptococcus productus, Clostridium ljungdahlii, Clostridium ATCC 29797* und *Clostridium carboxidivorans.*

10. Zelle nach einem der vorhergehenden Ansprüche, wobei es sich bei der Zelle um *Clostridium ljungdahlii* oder *Clostridium autothenogenum* DSMZ 10061 handelt.

11. Verfahren zur Herstellung von Acetoacetat, 3-Hydroxybutyrat und/oder 3-Hydroxybutyrat-Varianten, wobei das Verfahren
- Inkontaktbringen einer rekombinanten Mikrobenzelle nach einem der Ansprüche 1 bis 10 mit einem eine Kohlenstoffquelle umfassenden Medium
umfasst, wobei die 3-Hydroxybutyrat-Variante aus der Gruppe bestehend aus (S)-3-Hydroxybuttersäure, (S)-3-Hydroxybutyrat-Ester, (R)-3-Hydroxybuttersäure, (R)-3-Hydroxybutyrat-Ester, Natrium-3-hydroxybutyrat und 3-Hydroxybuttersäuremethylester ausgewählt ist.

12. Verfahren nach Anspruch 11, wobei die Kohlenstoffquelle CO₂ und/oder CO umfasst.

13. Verfahren nach entweder Anspruch 11 oder 12, wobei die Kohlenstoffquelle 50% oder mehr H₂ umfasst.

14. Verwendung der Zelle nach einem der Ansprüche 1 bis 10 zur Herstellung von Acetoacetat, 3-Hydroxybutyrat und/oder 3-Hydroxybutyrat-Varianten, wobei die 3-Hydroxybutyrat-Variante aus der Gruppe bestehend aus (S)-3-Hydroxybuttersäure, (S)-3-Hydroxybutyrat-Ester, (R)-3-Hydroxybuttersäure, (R)-3-Hydroxybutyrat-Ester, Natrium-3-hydroxybutyrat und 3-Hydroxybuttersäuremethylester ausgewählt ist.

## Revendications

1. Cellule microbienne qui est capable de produire de l'acétoacétate, du 3-hydroxybutyrate et/ou des variants du 3-hydroxybutyrate, la cellule étant génétiquement modifiée pour comprendre une expression accrue, par rapport à sa cellule de type sauvage, de :
- une enzyme E₁ étant capable de catalyser la conversion d'acétyl-CoA en acétoacétyl-CoA ;
- une enzyme E₂ étant capable de catalyser la conversion d'acétoacétyl-CoA en acétoacétate ; et
- une enzyme E₃ étant capable de catalyser la conversion d'acétoacétate en 3-hydroxybutyrate et/ou des variants de celui-ci, et la cellule génétiquement modifiée ayant une expression réduite ou nulle d'une acétoacétate décarboxylase (Adc ; EC 4.1.1.4),
dans laquelle le variant du 3-hydroxybutyrate est choisi dans le groupe constitué par l'acide (S)-3-hydroxybutyrique, l'ester (S)-3-hydroxybutyrate, l'acide (R)-3-hydroxybutyrique, l'ester (R)-3-hydroxybutyrate, le 3-hydroxybutyrate de sodium et le 3-hydroxybutyrate de méthyle.

2. Cellule selon la revendication 1, dans laquelle E₁ est une thiolase, E₂ est une acétoacétate CoA transférase et/ou E₃ est une déshydrogénase d'alcool secondaire.

3. Cellule selon l'une ou l'autre des revendications 1 ou 2, dans laquelle la thiolase (E₁) provient de *C*. *acetobutylicum.*

4. Cellule selon l'une quelconque des revendications précédentes, dans laquelle l'acétoacétate CoA transférase (E₂) provient de *C. acetobutylicum.*

5. Cellule selon l'une quelconque des revendications précédentes, dans laquelle la déshydrogénase d'alcool secondaire (E₃) provient de *C*. *beijerinckii.*

6. Cellule selon l'une quelconque des revendications précédentes, dans laquelle
- E₁ comprend une identité de séquence de 60% avec la SEQ ID n° : 2,
- E₂ comprend une identité de séquence de 60% avec les SEQ ID n° : 4 ou 6, et/ou
- E₃ comprend une identité de séquence de 60% avec la SEQ ID n° : 8.

7. Cellule selon l'une quelconque des revendications précédentes, dans laquelle
- E₁ comprend la SEQ ID n° : 2,
- E₂ comprend les SEQ ID n° : 4 et 6, et
- E₃ comprend la SEQ ID n° : 8.

8. Cellule selon l'une quelconque des revendications précédentes, la cellule étant une cellule acétogène.

9. Cellule selon la revendication 8, la cellule acétogène étant choisie dans le groupe constitué par *Clostridium autothenogenum DSMZ 19630, Clostridium ragsdahlei ATCC n° : BAA-622, Clostridium autoethanogenum, Moorella sp HUC22-1, Moorella thermoaceticum, Moorella thermoautotrophica, Rumicoccus productus, Acetoanaerobum, Oxobacter pfennigii, Methanosarcina barkeri, Methanosarcina acetivorans, Carboxydothermus, Desulfotomaculum kutznetsovii, Pyrococcus, Peptostreptococcus, Butyribacterium methylotrophicum ATCC 33266, Clostridium formicoaceticum, Clostridium butyricum, Lactobacillus delbrukii, Propionibacterium acidoproprionici, Proprionispera arboris, Anaerobierspirillum succiniproducens, Bacterioides amylophilus, Becterioides ruminicola, Thermoanaerobacter kivui, Acetobacterium woodii, Acetoanaerobium notera, Clostridium aceticum, Butyribacterium methylotrophicum, Moorella thermoacetica, Eubacterium limosum, Peptostreptococcus productus, Clostridium ljungdahlii, Clostridium ATCC 29797* et *Clostridium carboxidivorans.*

10. Cellule selon l'une quelconque des revendications précédentes, la cellule étant *Clostridium ljungdahlii* ou *Clostridium autothenogenum DSMZ 10061.*

11. Procédé de production d'acétoacétate, de 3-hydroxybutyrate et/ou de variants du 3-hydroxybutyrate, le procédé comprenant l'étape consistant à
- mettre en contact une cellule microbienne recombinante, selon l'une quelconque des revendications 1 à 10, avec un milieu comprenant une source de carbone dans laquelle le variant du 3-hydroxybutyrate est choisi dans le groupe constitué par l'acide (S)-3-hydroxybutyrique, l'ester (S)-3-hydroxybutyrate, l'acide (R)-3-hydroxybutyrique, l'ester (R)-3-hydroxybutyrate, le 3-hydroxybutyrate de sodium et le 3-hydroxybutyrate de méthyle.

12. Procédé selon la revendication 11, dans lequel la source de carbone comprend du CO₂ et/ou du CO.

13. Procédé selon l'une ou l'autre des revendications 11 ou 12, dans lequel la source de carbone comprend 50% ou plus de H₂.

14. Utilisation de la cellule, selon l'une quelconque des revendications 1 à 10, pour la production d'acétoacétate, de 3-hydroxybutyrate et/ou de variants du 3-hydroxybutyrate, dans laquelle le variant du 3-hydroxybutyrate est choisi dans le groupe constitué par l'acide (S)-3-hydroxybutyrique, l'ester (S)-3-hydroxybutyrate, l'acide (R)-3-hydroxybutyrique, l'ester (R)-3-hydroxybutyrate, le 3-hydroxybutyrate de sodium et le 3-hydroxybutyrate de méthyle.
